# EUROPEAN PATENT APPLICATION

(11) **EP 3 165 219 A1**
(43) Date of publication of application: **10.05.2017**
(21) Application number: 15814962.5
(22) Date of filing: 30.06.2015
(51) Int. Cl.: A61K 9/14, A61K 31/4178, A61K 47/32, A61K 47/38, A61K 47/34, A61P 9/12

(54) **ALLISARTAN ISOPROXIL SOLID DISPERSION AND PHARMACEUTICAL COMPOSITION COMPRISING SAME**

(30) Priority: 01.07.2014 CN 201410310848
(71) Applicant: Shenzhen Salubris Pharmaceuticals Co., Ltd, Shenzhen, Guangdong 518040 (CN)
(72) Inventor: YE, Guanhao, Shenzhen Guangdong 518040 (CN); BU, Shui, Shenzhen Guangdong 518040 (CN)
(74) Representative: Schollweck, Susanne
(86) International application number: PCT/CN2015/082994
(87) International publication number: WO 2016/000608

(57) **Abstract**

An allisartan isoproxil solid dispersion, pharmaceutical composition comprising the same and use thereof. The Allisartan Isoproxil solid dispersion consists of Allisartan Isoproxil and a pharmaceutically acceptable carrier material, the carrier material comprising a solubilizing carrier and a surfactant, the mass ratio of Allisartan Isoproxil to the surfactant being 1:0.01∼0.10.

## Description

### Field of the Invention

The present invention belongs to the field of pharmaceutical chemistry, in particular, it relates to allisartan isoproxil solid dispersion and pharmaceutical composition containing the solid dispersion.

### Background of the Invention

Allisartan isoproxil (CAS: 947331-05-7), with the chemical name: 2-butyl-4-chloro -1- [2 '- (1H- tetrazol-5-yl) -1, 1'-biphenyl - methyl] - imidazole-5-carboxylic acid, 1 - [(isopropoxy) - carbonyl oxy] - methyl ester, and the trade name: Xinlitan, is a novel angiotensin II receptor antagonist. Chinese patent CN200680000397.8 discloses the structure of allisartan isoproxil compound, which is with low toxicity, and better antihypertensive effect than products of the same type (such as losartan). It plays its antihypertensive effect by generating active metabolite (EXP3174) by metabolism in vivo. Allisartan isoproxil belongs to ester derivative of EXP3174 and its solubility in water is low, therefore, the resulting preparation using conventional preparation methods cannot meet the needs of clinical medication.

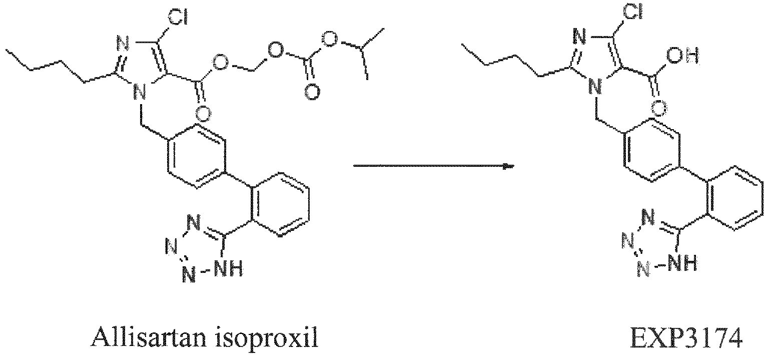

In order to overcome the low solubility of allisartan isoproxil, Chinese patent CN200880001668.0 provides a pharmaceutical composition of allisartan isoproxil.

By adding a specific carrier and using solid dispersion technology, the dissolution of active ingredient is effectively increased. Result of example D1-D6 show that dissolutions at 45 minutes are all more than 90% in line with the requirements of clinical medication. However, there are shortcomings in preparations of available technologies, e.g., drug loading in the mentioned solid dispersion is not high, so content of active ingredient in the pharmaceutical composition is low, and thus the mass of the preparation is too large, leading to poor patient compliance. It fails to achieve optimization of clinical administration composition mass and antihypertensive effect.

Therefore, improving the solid dispersion drug loading, and further reducing the mass of preparation units are technical problems to be solved first. However, there is technical difficulty to reduce the unit weight of formulation, it is known in the field, solid dispersion improves the dissolution of drug by dispersing active ingredient highly in carrier materials in the form of microcrystalline, amorphous or molecule.

However, affected by the formula or storage condition and other factors, the active ingredient in solid dispersion inevitably gathers or crystallizes in quickly or slowly rate during storage, which is named "ageing phenomenon" of solid dispersion.

Therefore, dissolution performance of preparations significantly decrease after a period of storage, even the initial dissolution are qualified. While reducing the amount of solid dispersion carrier, the aging phenomenon is particularly obvious. The conclusion can be drawn that the speed and extent of the solid dispersion ageing is an important factor in quality evaluation of solid dispersion, and the key consideration in order to improve the drug loading.

Therefore, in premise of ensuring the dissolution of the formulation while avoiding the occurrence of ageing phenomenon, improving the content of the active ingredient in solid dispersion, and therefor reducing the unit weight of the preparation and improve patient's compliance with medication is unresolved problems in available technologies. Starts from solving the shortcomings of available technologies, the present invention provides a new allisartan isoproxil solid dispersion with high stability and good dissolution performance after a large number of experiments. The mentioned solid dispersion reduces the amount of carrier and improves the drug loading by the addition of surfactant, thus also increases the content of active ingredient in the preparation, and reduces the unit weight of preparation.

### Summary of the Invention

The object of the present invention is to overcome the shortcomings of available technologies. In the premise of ensuring the stability and dissolution of the preparation, active ingredient content in the solid dispersion is effectively increased through the addition of surfactants; further a solid dispersion of allisartan isoproxil with high drug loading is discovered. The drug loading of the solid dispersion is higher than that of the available technologies, and pharmaceutical composition containing the mentioned solid dispersion shows good dissolution property, high stability, etc. which meets the requirements of clinical medication. Finally clinical administration composition mass and antihypertensive effect relationships are optimized with improved patient compliance.

The above-described advantages of the mentioned solid dispersions are achieved by the following technical means:
An allisartan isoproxil solid dispersion is comprosed of allisartan isoproxil and pharmaceutically acceptable carrier material. The mentioned carrier material comprises solubilizing carrier, wherein allisartan isoproxil solid dispersion also comprises surfactant, and the mass ratio of allisartan isoproxil to the surfactant is 1: 0.01 to 0.10.

It is known in this field, solid dispersions refer to disperse active ingredient in carrier materials in the form of microcrystalline, amorphous or molecule through preparation methods, thereby improving the dissolution of drug. However, when the preparation methods cannot make the obtained formulation meet the requirements of clinical use, further solubilizing excipient should be added to enhance drug dissolution. The surfactant is one kind of the commonly used solubilizing pharmaceutical excipients.

It is known in this field that the surfactant refers to a class of substances with strong surfactivity which can significantly reduce the surface tension of the liquid. According to the dissociation nature of the polar group, surfactants commonly used in the preparation field include anionic surfactants, cationic surfactants, zwitterionic surfactants and nonionic surfactants. For this invention, during the preparation process, the inventor has found that addition of an appropriate amount of surfactant can improve the dissolution of the formulation and reduce the use of solubilizing carrier. More surprisingly, the inventor has found through experiments, for allisartan isoproxil compound, the amount of surfactant also can improve the stability of the solid dispersion. Specifically, the use of surfactant can stabilize the solid dispersion and slow down the speed of ageing. For this case, in order to achieve the desired technical effect, the surfactant is selected from one or mixture of two or more of anionic surfactants, such as sodium lauryl sulfate, hexadecyl sulfate, octadecyl sulfate; zwitterionic surfactants, such as lecithin (soy lecithin, egg yolk lecithin); nonionic surfactants such as polyol type (sucrose stearate), fatty acid sorbitan (Span 20, 40, 60, 80), polysorbate (Tween 20, 40, 60, 80), polyoxyethylene fatty alcohol ethers (polyoxyethylene 35 castor oil, polyoxyl 40 hydrogenated castor oil), preferably one or mixture of two or more of sodium dodecyl sulfate, lecithin (soy lecithin, egg yolk lecithin), sucrose stearate, span 20, Tween 20, Tween 80, Polyoxyl 40 Hydrogenated Castor Oil, etc..

For allisartan isoproxil product, there is certain correlation between the amount of surfactant and drug loading & stability of solid dispersion. Drug loading, i.e. the amount of drug loading, refers to the loading amount of the active ingredient in unit weight of carrier of the solid dispersion. The higher the loading dose in the solid dispersion, the more difficult to achieve. Specifically, in the present invention, certain amount of surfactant adding can significantly improve dissolution, reduce the amount of the carrier, and avoid ageing phenomenon, thus increase the drug loading in solid dispersion and reduce the unit weight of preparation; however, with the increase of the amount of surfactant, its effect on improving drug loading and dissolution is gradually weaken, after reaching a certain degree, it will reduce the amount of drug loading. The inventor conducted a large number of screening experiments, and it is found, when the mass ratio of Allisartan isoproxil to surfactant is 1: 0.01 to 0.10, preferably 1: 0.02 to 0.06, the allisartan isoproxil solid dispersion shows better stability and dissolution, and the drug loading of the solid dispersion can be significantly improved.

According to the present invention, pharmaceutically acceptable carrier material contains solubilizing carrier. Generally, more solid dispersion carrier material in solid dispersion help increase active ingredient dispersed uniformly and stably, so as to achieve the purpose of improving drug dissolution, and the difficulty of preparation of solid dispersion is lower correspondingly. In the solid dispersion of the present invention, the amount of carrier material can achieve the drug loading effect is well known to the person skilled in the art. However, with the addition of surfactant, the amount of carrier is significantly reduced in the solid dispersion on the basis of ensuring the stability, thereby the drug loading of solid dispersion is increased.

The mentioned solubilizing carrier in present invention means the pharmaceutical excipient which can play the role of dispersion, and refers to a series of pharmaceutical excipients with solubilization, specifically, the solubilizing carrier is selected from one or mixture in any ratio of two or more of povidone, copovidone and other vinyl pyrrolidone homopolymers or copolymers; polyvinyl alcohol; polyethylene glycol (PEG4000, PEG6000); methylcellulose, hydroxypropyl cellulose, hydroxypropylmethyl cellulose and other cellulose ethers; Eugragit L 100 and S100, acrylic resin II, acrylic resin III and other acrylic polymers; hydroxypropyl methyl cellulose phthalate (HPMCP HP-55), cellulose acetate phthalate (CAP), hydroxypropyl methyl cellulose acetate succinate (HPMCAS), preferably one or mixture in any ratio of two or more of povidone, copovidone, polyethylene glycol (PEG4000, PEG6000), hydroxypropyl cellulose, hydroxypropylmethyl cellulose, hydroxypropylmethyl cellulose phthalate (HPMCP HP-55). Despite increasing the amount of solubilizing carrier is beneficial on preparing solid dispersions, it also reduces the drug load of the solid dispersion correspondingly. In the premise of adding a surfactant, the amount of solubilizing carrier can be significantly reduced in allisartan isoproxil solid dispersion, but too low amount of solubilizing carrier cannot achieve its dispersion effect, and thus cannot form solid dispersion. More specifically, the inventor conducts a large number of experiments and find that when the mass ratio of allisartan isoproxil and solubilizing carrier is 1: 0.15 to 0.5, preferably 1: 0.20 to 0.30, it's good for achieving the optimization of drug load and quality of solid dispersion.

Wherein the mentioned povidone is 1-vinyl-2-pyrrolidinone homopolymer.

According to average molecular weight, the specifications include PVP k12, PVP k15, PVP k17, PVP k25, PVP k30, PVP k29 / 32, PVP k60, PVP k120, etc., preferably PVP k29 / 32. The mentioned hydroxypropyl cellulose is classified according to the average molecular weight as HPC-SSL, HPC-SL, HPC-L, HPC-M, HPC-H, etc., preferably HPC-SL. The mentioned hydroxypropyl methyl cellulose is classified according to viscosity as E3, E5, E6, E15, E50Lv, etc., preferably HPMC E6.

Further, depending on the requirement of formulation and preparation method, the carrier material of the allisartan isoproxil solid dispersion can further include excipient, the mentioned excipient play role in carrying and assisting the dispersion of active ingredient, too little excipient cannot play the role of carrying and dispersion, while too much will make the mass of preparation too large, also make the preparation process complicated. Specifically, the excipient can be select from one or mixture in any ratio of more of disintegrant, filler, binder and other pharmaceutical excipient except solubilization carrier and surfactant, more specifically, the excipient is selected from one or mixture in any ratio of two or more of cross-linked povidone, cross-linked sodium carboxymethyl cellulose, low-substituted hydroxypropyl cellulose, sodium carboxymethyl starch, microcrystalline cellulose, starch, pre-gelatinized starch, lactose, dextrin, mannitol, calcium sulfate, calcium phosphate, calcium hydrogen phosphate and other pharmaceutical excipient, preferably cross-linked povidone. The mentioned cross-linked povidone is synthetic crosslinked N- vinyl-2-pyrrolidone homopolymer, such as PVPP XL, PVPP XL-10. The mass ratio of allisartan isoproxil to excipient is 1: 0.10 ∼ 1.00, preferably 1: 0.30 ∼ 0.80.

The surfactant, solubilizing carrier and excipient can optionally be selected from the above-described species, e.g., a preferred technical solution of the present invention, the solid dispersion has the following compositions:

| Components | Content (unit) |
|---|---|
| Allisartan isoproxil | 1 |
| Sodium dodecyl sulfate | 0.01 |
| Egg yolk lecithin | 0.01 |
| Povidone k29/32 | 0.35 |
| Crosslinked povidone | 0.35 |

A preferred technical solution of the solid dispersion in the present invention, and the compositions are as follows:

| Components | Content (unit) |
|---|---|
| Allisartan isoproxil | 1 |
| Sodium dodecyl sulfate | 0.025 |
| Povidone k29/32 | 0.20 |
| Crosslinked povidone | 0.40 |

A preferred technical solution of the solid dispersion in the present invention, and the compositions are as follows:

| Components | Content (unit) |
|---|---|
| Allisartan isoproxil | 1 |
| Egg yolk lecithin | 0.05 |
| Povidone k29/32 | 0.30 |
| Microcrystalline cellulose | 0.30 |
| Crosslinked povidone | 0.05 |

A preferred technical solution of the solid dispersion in the present invention, and the compositions are as follows:

| Components | Content (unit) |
|---|---|
| Allisartan isoproxil | 1 |
| Soy lecithin | 0.05 |
| PEG6000 | 0.25 |
| Sodium carboxymethyl starch (I) | 0.10 |
| Microcrystalline cellulose | 0.40 |

A preferred technical solution of the solid dispersion in the present invention, and the compositions are as follows:

| Components | Content (unit) |
|---|---|
| Allisartan isoproxil | 1 |
| Tween 20 | 0.02 |
| Copovidone S630 | 0.35 |
| Microcrystalline cellulose | 0.45 |

A preferred technical solution of the solid dispersion in the present invention, and the compositions are as follows:

| Components | Content (unit) |
|---|---|
| Allisartan isoproxil | 1 |
| Sucrose stearate | 0.06 |
| Hydroxypropyl cellulose SL | 0.375 |
| Microcrystalline cellulose | 0.37 |

A preferred technical solution of the solid dispersion in the present invention, and the compositions are as follows:

| Components | Content (unit) |
|---|---|
| Allisartan isoproxil | 1 |
| Polyoxyl 40 Hydrogenated Castor Oil | 0.035 |
| HPMCP HP-55 | 0.40 |
| Crosslinked povidone | 0.35 |

A preferred technical solution of the solid dispersion in the present invention, and the compositions are as follows:

| Components | Content (unit) |
|---|---|
| Allisartan isoproxil | 1 |
| Span 20 | 0.055 |
| Povidone k29/32 | 0.20 |
| Crosslinked povidone | 0.40 |

The allisartan isoproxil solid dispersion can be prepared using conventional manufacturing methods in this field depending on the specific formulations, such as solvent method, solvent deposition method, spray drying method, fluidized bed method, freeze drying method, preferably fluidized bed method.

For a preferred aspect of the present invention specification, preparation process of allisartan isoproxil solid dispersion comprises the following steps:
1. Dissolve the allisartan isoproxil and solubilizing carrier in an appropriate amount of organic solvent, add a surfactant and then mix to obtain drug solution;
2. Place the excipient in the fluidized bed, and spray drug solution from top to perform fluidized bed dried (FBD) granulation, and get allisartan isoproxil solid dispersion.

It is another object of the present invention to provide a pharmaceutical composition of allisartan isoproxil. The mentioned pharmaceutical composition is composed of allisartan isoproxil solid dispersion of the present invention and pharmaceutical excipient. Depending on the requirement of formulation, the pharmaceutical excipient can be selected from one or mixture of two or more of disintegrant, binder, filler, lubricant, etc.

The mentioned disintegrant is selected from one or mixture of two or more of cross-linked sodium carboxymethyl cellulose, dry starch, crosslinked povidone, sodium carboxymethyl starch, low-substituted hydroxypropyl cellulose, starch, pregelatinized starch, etc.; the amount of disintegrant should be known in this field can achieve the effect of disintegrating, for allisartan isoproxil pharmaceutical compositions, using too much disintegrant is adverse to control the unit mass of the preparation, while using too little of the disintegrant will cause long time disintegration, so that dissolution of the corresponding pharmaceutical composition cannot meet the requirements of clinical use. Preferably, the mass ratio of the solid dispersion to disintegrant in the pharmaceutical composition is 1: 0.02 to 0.20.

The mentioned binder can be added depending on the needs of pharmaceutical formulations; specifically, the binder is selected from one or mixture of two or more of hydroxypropyl methylcellulose, hydroxypropyl cellulose, sodium carboxymethyl cellulose, povidone, starch slurry, gelatin, etc. When adding the binder, the amount should be known in this field can achieve the effect of adhesive, preferably, the mass ratio between solid dispersion to the binder in pharmaceutical composition is 1: 0.01 to 0.05.

The mentioned filler can be added depending on the need of pharmaceutical formulations, specifically, the filler is selected from one or mixture of two or more of lactose, mannitol, dextrin, microcrystalline cellulose, starch, pregelatinized starch, calcium sulfate, calcium phosphate, calcium hydrogen phosphate, etc. The amount of filler should be known in this field can achieve the filling effect; preferably, the mass ratio of solid dispersion to the filler in pharmaceutical composition is 1: 0.02 to 0.20.

The mentioned lubricant is selected from one or mixture of two or more of stearic acid, magnesium stearate, colloidal silicon dioxide, talc, polyethylene glycol, etc. The amount of lubricant should be known in this field can achieve lubricating effect.

The pharmaceutical compositions of allisartan isoproxil can be tablets, capsules, granules, pills and other conventional oral preparations, preferably tablets and capsules.

The pharmaceutical compositions should be prepared using common preparation means in this field, specifically, for tablets, dry granulation, wet granulation and direct compression method can be used; for capsules, dry granulation, wet granulation or direct-powder-fill method can be used.

For one embodiment of allisartan isoproxil pharmaceutical composition in the present invention, the formulation and preparation method are as follows:

| Components | Content (unit) |
|---|---|
| Solid dispersion | 1 |
| Crosslinked povidone | 0.09 |
| Magnesium stearate | 0.01 |
| Opadry | 0.02 |

Mix the solid dispersion obtained and excipients, then directly compress into tablets. If necessary, it can be further coated to obtain coated tablets.

The allisartan isoproxil pharmaceutical composition in present invention can be used for the treatment of hypertension and its complications, preferably, mentioned allisartan isoproxil pharmaceutical composition can be used for mild to moderate primary hypertension treatment. The complications of hypertension refer to symptoms caused by hypertension, including cardiac complications, such as left ventricular hypertrophy, angina, myocardial infarction, heart failure; stroke, such as hemorrhagic stroke, ischemic stroke, hypertensive encephalopathy; hypertensive renal damage, such as the slow progress of the small arteries of the kidney sclerosis, malignant small renal arterial sclerosis, chronic renal failure; ophthalmic diseases, such as retinal arteriosclerosis, retinal change.

The present invention shows following outstanding advantages and beneficial effects compared with available technologies:
1. Provides an allisartan isoproxil solid dispersion with high drug loading by adding surfactant, so that the solid dispersion has obvious advantages over available technologies in drug loading, dissolution, stability and so on;
2. Provides a pharmaceutical composition of allisartan isoproxil containing allisartan isoproxil solid dispersion mentioned in the present invention, shows good dissolution performance and high stability. It finally optimizes the clinical dose and the antihypertensive effect.

### Detailed description of the Examples

As below the present invention will be described in further detail in conjunction with examples, but it is not limited to this.

### Example 1

### Formulation:

| Type | Components | Content (mg/tablet) |
|---|---|---|
| Solid dispersion | Allisartan isoproxil | 240 |
| | Sodium lauryl sulfate | 2.4 |
| | Egg yolk lecithin | 2.4 |
| | Povidonek29/32 | 84 |
| | Crosslinked povidone(I) | 84 |
| Extragranular material | Microcrystalline cellulose | 36 |
| | Crosslinked povidone(II) | 36 |
| | Magnesium stearate | 4.1 |
| Coating material | Opadry | 9.8 |
| Theoretical tablet weight | | 498.7 |

### Preparation:

### 1. Preparation of solid dispersion

Dissolved the drug and povidone K29/32 in a suitable amount of methylene chloride-ethanol mixed solution firstly, and then added sodium lauryl sulfate and egg yolk lecithin solution inside, finally mixed to be even. Put crosslinked povidone (I) into the fluidized bed, sprayed the solution prepared into fluidized bed granulator from top using a spray gun, and dried to obtain allisartan isoproxil solid dispersion; further XRD testing showed that allisartan isoproxil was highly dispersed in the solid dispersion, proving the desired effect was achieved.

### 2. Preparation of the pharmaceutical composition

Mixed the solid dispersion with the remaining extragranular materials, compressed into tablets, performed film coating and finally obtained an Allisartan isoproxil pharmaceutical composition.

### Example 2

### Formulation:

| Type | Components | Content (mg/tablet) |
|---|---|---|
| Solid dispersion | Allisartan isoproxil | 240 |
| | Sodium lauryl sulfate | 6.0 |
| | Povidonek29/32 | 48 |
| | Crosslinked povidone (I) | 96 |
| Extragranular material | Microcrystalline cellulose | 37.2 |
| | Lactose | 11.2 |
| | Crosslinked povidone (II) | 11.2 |
| | Magnesium stearate | 3.9 |
| Coating material | Opadry | 9.1 |
| Theoretical tablet weight | | 462.6 |

### Preparation:

### 1. Preparation of solid dispersion

Dissolved the drug and povidone K29/32 in a suitable amount of methylene chloride-ethanol mixed solution firstly, and then added sodium lauryl sulfate solution inside, finally mixed to be even. Put crosslinked povidone (I) into the fluidized bed, sprayed the solution prepared into a fluidized bed granulator from top using a spray gun, and dried to obtain allisartan isoproxil solid dispersion; further XRD testing showed that allisartan isoproxil was highly dispersed in the solid dispersion, proving the desired effect was achieved.

### 2. Preparation of the pharmaceutical composition

Mixed the solid dispersion with the remaining extragranular materials, compressed into tablets, performed film coating and finally obtained an allisartan isoproxil pharmaceutical composition.

### Example 3

### Formulation:

| Type | Components | Content (mg/tablet) |
|---|---|---|
| Solid dispersion | Allisartan isoproxil | 240 |
| | Egg yolk lecithin | 12 |
| | Povidonek29/32 | 72 |
| | Microcrystalline cellulose | 72 |
| | Crosslinked povidone (I) | 12 |
| Extragranular material | Crosslinked povidone (II) | 37.2 |
| | Magnesium stearate | 4.1 |
| Coating material | Opadry | 9.0 |
| Theoretical tablet weight | | 458.3 |

### Preparation:

### 1. Preparation of solid dispersion

Dissolved the drug and povidone K29/32 in a suitable amount of methylene chloride - ethanol mixed solution firstly, then added egg yolk lecithin inside, dissolved and finally mixed to be even. Put microcrystalline cellulose and crosslinked povidone (I) into the fluidized bed, sprayed the solution prepared into a fluidized bed granulator from top using a spray gun, and dried to obtain allisartan isoproxil solid dispersion; further XRD testing showed that allisartan isoproxil was highly dispersed in the solid dispersion, proving the desired effect was achieved.

### 2. Preparation of the pharmaceutical composition

Mixed the solid dispersion with the remaining extragranular materials, compressed into tablets, performed film coating and finally obtained an allisartan isoproxil pharmaceutical composition.

### Example 4

### Formulation:

| Type | Components | Content (mg/tablet) |
|---|---|---|
| Solid dispersion | Allisartan isoproxil | 240 |
| | Soybean lecithin | 12 |
| | PEG6000 | 60 |
| | Sodium carboxymethyl starch (I) | 24 |
| | Microcrystalline cellulose | 96 |
| Extragranular material | Sodium carboxymethyl starch (II) | 48 |
| | Magnesium stearate | 4.3 |
| Coating material | Opadry | 9.7 |
| Theoretical tablet weight | | 494.0 |

### Preparation:

### 1. Preparation of solid dispersion

Dissolved the drug and PEG6000 in a suitable amount of methylene chloride-ethanol mixed solution firstly, then added soybean lecithin inside, dissolved, and finally mixed to be even. Put microcrystalline cellulose and sodium carboxymethyl starch (I) into the fluidized bed, sprayed the solution prepared into a fluidized bed granulator from top using a spray gun, and dried to obtain allisartan isoproxil solid dispersion; further XRD testing showed that allisartan isoproxil was highly dispersed in the solid dispersion, proving the desired effect was achieved.

### 2. Preparation of the pharmaceutical composition

Mixed the solid dispersion with the remaining extragranular materials, compressed into tablets, performed film coating and finally obtained an allisartan isoproxil pharmaceutical composition.

### Example 5

### Formulation:

| Type | Components | Content (mg/tablet) |
|---|---|---|
| Solid dispersion | Allisartan isoproxil | 240 |
| | Tween20 | 4.8 |
| | Copovidone S630 | 84 |
| | Microcrystalline cellulose | 108 |
| Extragranula r material | Crosslinked povidone | 55 |
| | Magnesium stearate | 4.4 |
| Coating material | Opadry | 9.9 |
| Theoretical tablet weight | | 506.1 |

### Preparation:

### 1. Preparation of solid dispersion

Dissolved the drug and copovidone S630 in a suitable amount of methylene chloride-ethanol mixed solution firstly, then added tween 20 inside, stirred, and finally mixed to be even. Put microcrystalline cellulose into the fluidized bed, sprayed the solution prepared into a fluidized bed granulator from top using a spray gun, and dried to obtain allisartan isoproxil solid dispersion; further XRD testing showed that allisartan isoproxil was highly dispersed in the solid dispersion, proving the desired effect was achieved.

### 2. Preparation of the pharmaceutical composition

Mixed the solid dispersion with the remaining extragranular materials, compressed into tablets, performed film coating and finally obtained an allisartan isoproxil pharmaceutical composition.

### Example 6

### Formulation:

| Type | Components | Content (mg/tablet) |
|---|---|---|
| Solid dispersion | Allisartan isoproxil | 240 |
| | Sucrose stearate | 14.4 |
| | Hydroxy propyl cellulose SL | 90 |
| | Microcrystalline cellulose | 89 |
| Extragranular material | Low-substituted hydroxypropyl cellulose | 52 |
| | Magnesium stearate | 4.3 |
| Coating material | Opadry | 9.8 |
| Theoretical tablet weight | | 499.5 |

### Preparation:

### 1. Preparation of solid dispersion

Dissolved the drug and hydroxy propyl cellulose SL in a suitable amount of methylene chloride-ethanol mixed solution firstly, then added sucrose stearate inside, dissolved, and finally mixed to be even. Put microcrystalline cellulose into the fluidized bed, sprayed the solution prepared into a fluidized bed granulator from top using a spray gun, and dried to obtain allisartan isoproxil solid dispersion; further XRD testing showed that allisartan isoproxil was highly dispersed in the solid dispersion, proving the desired effect was achieved.

### 2. Preparation of the pharmaceutical composition

Mixed the solid dispersion with the remaining extragranular materials, compressed into tablets, performed film coating and finally obtained an allisartan isoproxil pharmaceutical composition.

### Example 7

### Formulation:

| Type | Components | Content (mg/tablet) |
|---|---|---|
| Solid dispersion | Allisartan isoproxil | 240 |
| | Polyoxyl 40 Hydrogenated Castor Oil | 8.3 |
| | HPMCP HP-55 | 96 |
| | Crosslinked povidone (I) | 84 |
| Extragranular material | Crosslinked povidone (II) | 25 |
| | Microcrystalline cellulose | 25 |
| | Povidonek29/32 | 5.0 |
| | Magnesium stearate | 4.3 |
| Coating material | Opadry | 9.7 |
| Theoretical tablet weight | | 497.3 |

### Preparation:

### 1. Preparation of solid dispersion

Dissolved the drug and HPMCP HP-55 in a suitable amount of methylene chloride - ethanol mixed solution firstly, then added Polyoxyl 40 Hydrogenated Castor Oil inside, stirred, and finally mixed to be even. Put crosslinked povidone (I) into the fluidized bed, sprayed the solution prepared into a fluidized bed granulator from top using a spray gun, and dried to obtain allisartan isoproxil solid dispersion; further XRD testing showed that allisartan isoproxil was highly dispersed in the solid dispersion, proving the desired effect was achieved.

### 2. Preparation of the pharmaceutical composition

Mixed the solid dispersion with the remaining extragranular materials, compressed into tablets, performed film coating and finally obtained an allisartan isoproxil pharmaceutical composition.

### Example 8

### Formulation:

| Type | Components | Content (mg/tablet) |
|---|---|---|
| Solid dispersion | Allisartan isoproxil | 240 |
| | Span 20 | 13.2 |
| | Povidone k29/32 | 48 |
| | Crosslinked povidone (I) | 96 |
| Extragranular material | Crosslinked povidone (II) | 11.2 |
| | Lactose | 37.2 |
| | Stearic acid | 4.0 |
| Coating material | Opadry | 9.0 |
| Theoretical tablet weight | | 458.6 |

### Preparation:

### 1. Preparation of solid dispersion

Dissolved the drug and povidone K29/32 in a suitable amount of methylene chloride - ethanol mixed solution firstly, then added span 20 inside, stirred, and finally mixed to be even. Put crosslinked povidone (I) into the fluidized bed, sprayed the solution prepared into a fluidized bed granulator from top using a spray gun, and dried to obtain allisartan isoproxil solid dispersion; further XRD testing showed that allisartan isoproxil was highly dispersed in the solid dispersion, proving the desired effect was achieved.

### 2. Preparation of the pharmaceutical composition

Mixed the solid dispersion with the remaining extragranular materials, compressed into tablets, performed film coating and finally obtained an allisartan isoproxil pharmaceutical composition.

### Example 9

### Formulation:

| Type | Components | Content (mg/tablet) |
|---|---|---|
| Solid dispersion | Allisartan isoproxil | 240 |
| | Octadecyl sodium sulfate | 3.2 |
| | Povidone k29/32 | 48 |
| | Crosslinked povidone (I) | 96 |
| Extragranular material | Microcrystalline cellulose | 37.2 |
| | Lactose | 11.2 |
| | Crosslinked povidone (II) | 11.2 |
| | Colloidal silicon dioxide | 3.9 |
| Coating material | Opadry | 9.1 |
| Theoretical tablet weight | | 459.8 |

### Preparation:

### 1. Preparation of solid dispersion

Dissolved the drug and povidone K29/32 in a suitable amount of methylene chloride - ethanol mixed solution firstly, then added octadecyl sodium sulfate inside, stirred, and finally mixed to be even. Put crosslinked povidone (I) into the fluidized bed, sprayed the solution prepared into a fluidized bed granulator from top using a spray gun, and dried to obtain allisartan isoproxil solid dispersion; further XRD testing showed that allisartan isoproxil was highly dispersed in the solid dispersion, proving the desired effect was achieved.

### 2. Preparation of the pharmaceutical composition

Mixed the solid dispersion with the remaining extragranular materials, compressed into tablets, performed film coating and finally obtained an allisartan isoproxil pharmaceutical composition.

### Example 10

### Formulation:

| Type | Components | Content (mg/tablet) |
|---|---|---|
| Solid dispersion | Allisartan isoproxil | 240 |
| | Cetyl sodium sulfate | 21 |
| | Povidone k29/32 | 72 |
| | Microcrystalline cellulose | 72 |
| | Crosslinked povidone (I) | 12 |
| Extragranular material | Crosslinked povidone (II) | 37.2 |
| | Magnesium stearate | 4.1 |
| Coating material | Opadry | 9.0 |
| Theoretical tablet weight | | 458.3 |

### Preparation:

### 1. Preparation of solid dispersion

Dissolved the drug and povidone K29/32 in a suitable amount of methylene chloride - ethanol mixed solution firstly, then added cetyl sodium sulfate inside, stirred, and finally mixed to be even. Put microcrystalline cellulose and crosslinked povidone (I) into the fluidized bed, sprayed the solution prepared into a fluidized bed granulator from top using a spray gun, and dried to obtain allisartan isoproxil solid dispersion; further XRD testing showed that allisartan isoproxil was highly dispersed in the solid dispersion, proving the desired effect was achieved.

### 2. Preparation of the pharmaceutical composition

Mixed the solid dispersion with the remaining extragranular materials, compressed into tablets, performed film coating and finally obtained an allisartan isoproxil pharmaceutical composition.

### Comparative Example 1

### Formulation:

| Type | Components | Content (mg/tablet) |
|---|---|---|
| Solid dispersion | Allisartan isoproxil | 240 |
| | Povidone k29/32 | 128 |
| | Crosslinked povidone (I) | 320 |
| Extragranular material | Crosslinked povidone (II) | 40 |
| | Lactose | 160 |
| | Stearic acid | 6.4 |
| Coating material | Opadry | 17.9 |
| Theoretical tablet weight | | 912.3 |

### Preparation:

### 1. Preparation of solid dispersion

Dissolved the drug and povidone K29/32 in a suitable amount of methylene chloride - ethanol mixed solution firstly, then put crosslinked povidone (I) into the fluidized bed, sprayed the solution prepared into a fluidized bed granulator from top using a spray gun, and dried to obtain allisartan isoproxil solid dispersion; further XRD testing showed that allisartan isoproxil was highly dispersed in the solid dispersion, proving the desired effect was achieved.

### 2. Preparation of the pharmaceutical composition

Mixed the solid dispersion with the remaining extragranular materials, compressed into tablets, performed film coating and finally obtained an allisartan isoproxil pharmaceutical composition.

### Comparative Example 2

### Formulation:

| Type | Components | Content (mg/tablet) |
|---|---|---|
| Solid dispersion | Allisartan isoproxil | 240 |
| | Povidone k29/32 | 72 |
| | Microcrystalline cellulose | 72 |
| | Crosslinked povidone (I) | 12 |
| Extragranular material | Crosslinked povidone (II) | 37.2 |
| | Magnesium stearate | 4.1 |
| Coating material | Opadry | 9.0 |
| Theoretical tablet weight | | 446.3 |

### Preparation:

### 1. Preparation of solid dispersion

Dissolved the drug and povidone K29/32 in a suitable amount of methylene chloride-ethanol mixed solution firstly, then put microcrystalline cellulose and crosslinked povidone (I) into the fluidized bed, sprayed the solution prepared into a fluidized bed granulator from top using a spray gun, and dried to obtain allisartan isoproxil solid dispersion; further XRD testing showed that allisartan isoproxil was highly dispersed in the solid dispersion, proving the desired effect was achieved.

### 2. Preparation of the pharmaceutical composition

Mixed the solid dispersion with the remaining extragranular materials, compressed into tablets, performed film coating and finally obtained an allisartan isoproxil pharmaceutical composition.

### Comparative Example 3

### Formulation:

| Type | Components | Content (mg/tablet) |
|---|---|---|
| Solid dispersion | Allisartan isoproxil | 240 |
| | Poloxamer188 | 12.0 |
| | Povidone k29/32 | 72 |
| | Microcrystalline cellulose | 72 |
| | Crosslinked povidone (I) | 12 |
| Extragranular material | Crosslinked povidone (II) | 37.2 |
| | Magnesium stearate | 4.1 |
| Coating material | Opadry | 9.0 |
| Theoretical tablet weight | | 458.3 |

### Preparation:

### 1. Preparation of solid dispersion

Dissolved the drug and povidone K29/32 in a suitable amount of methylene chloride-ethanol mixed solution firstly, then added poloxamer 188 inside, dissolved and mixed to be even. Put microcrystalline cellulose and crosslinked povidone (I) into the fluidized bed, sprayed the solution prepared into a fluidized bed granulator from top using a spray gun, and dried to obtain allisartan isoproxil solid dispersion; further XRD testing showed that allisartan isoproxil was highly dispersed in the solid dispersion, proving the desired effect was achieved.

### 2. Preparation of the pharmaceutical composition

Mixed the solid dispersion with the remaining extragranular materials, compressed into tablets, performed film coating and finally obtained an allisartan isoproxil pharmaceutical composition.

### Comparative Example 4

### Formulation:

| Type | Components | Content (mg/tablet) |
|---|---|---|
| Solid dispersion | Allisartan isoproxil | 240 |
| | Tween20 | 1.2 |
| | Copovidone S630 | 84 |
| | Microcrystalline cellulose | 108 |
| Extragranular material | Crosslinked povidone | 55 |
| | Magnesium stearate | 4.4 |
| Coating material | Opadry | 9.9 |
| Theoretical tablet weight | | 502.5 |

### Preparation:

### 1. Preparation of solid dispersion

Dissolved the drug and copovidone in a suitable amount of methylene chloride-ethanol mixed solution firstly, then added Tween 20 inside, dissolved and mixed to be even. Put microcrystalline cellulose into the fluidized bed, sprayed the solution prepared into a fluidized bed granulator from top using a spray gun, and dried to obtain allisartan isoproxil solid dispersion; further XRD testing showed that allisartan isoproxil was highly dispersed in the solid dispersion, proving the desired effect was achieved.

### 2. Preparation of the pharmaceutical composition

Mixed the solid dispersion with the remaining extragranular materials, compressed into tablets, performed film coating and finally obtained an allisartan isoproxil pharmaceutical composition.

### Example 11

Tested according to the second method in Appendix XC of the Chinese Pharmacopoeia (2010 Edition) in the dissolution test. Tested the dissolution of allisartan isoproxil pharmaceutical compositions obtained in Example 1 to 10 and Comparative Example 1 to 4 at 0 day in pH6.8 phosphate buffer (37 °C, dissolution medium 900mL, speed 50rpm) respectively. Sampled at 15min, 30min and 45min. Tested by UV spectrophotometer.

| Example | 15min | 30min | 45min |
|---|---|---|---|
| Example 1 | 87% | 93% | 95% |
| Example 2 | 90% | 94% | 95% |
| Example 3 | 93% | 98% | 98% |
| Example 4 | 91% | 95% | 96% |
| Example 5 | 87% | 92% | 95% |
| Example 6 | 86% | 93% | 95% |
| Example 7 | 85% | 92% | 95% |
| Example 8 | 90% | 97% | 97% |
| Example 9 | 88% | 93% | 94% |
| Example 10 | 90% | 95% | 98% |
| Comparative Example 1 | 85% | 93% | 96% |
| Comparative Example 2 | 86% | 90% | 96% |
| Comparative Example 3 | 64% | 73% | 75% |
| Comparative Example 4 | 84% | 90% | 92% |

As could be seen from the above data, allisartan isoproxil pharmaceutical compositions obtained in Example 1 to 10 and Comparative Example 1, 2 and 4 could reach the dissolution of 90% or more in 45 minutes. Comparative Example 2 had the same active ingredient & excipients and related amount with Example 3 except that no surfactant mentioned the present invention was added, but its dissolution in 45 minutes was lower than that of Example 3. In Comparative Example 4, same amount active ingredient & excipients to Example 5 but less surfactant mentioned in the present invention was added, and its dissolution in 45 minutes was lower than that of Example 5. In Comparative Example 3, since the surfactant added wasn't ones mentioned in the present invention, its dissolution decreased, which could not meet the requirement of clinical use.

It could be seen from the above results that addition of surfactant in the present invention could improve dissolution of allisartan isoproxil pharmaceutical composition.

### Example 12

Stored Allisartan isoproxil pharmaceutical compositions obtained in Example 1 to 10 and Comparative Example 1 to 4 at 40 °C for 30 days, and tested the dissolution by same method as described in Example 11.

| Example | 15min | 30min | 45min |
|---|---|---|---|
| Example 1 | 85 % | 96% | 96% |
| Example 2 | 89% | 95% | 95% |
| Example 3 | 95% | 97% | 98% |
| Example 4 | 90% | 95% | 96% |
| Example 5 | 83% | 89% | 91% |
| Example 6 | 85% | 92% | 95% |
| Example 7 | 83% | 90% | 93% |
| Example 8 | 89% | 95% | 96% |
| Example 9 | 84% | 87% | 91% |
| Example 10 | 88% | 95% | 97% |
| Comparative Example 1 | 83% | 92% | 96% |
| Comparative Example 2 | 55% | 60% | 63% |
| Comparative Example 3 | 50% | 55% | 56% |
| Comparative Example 4 | 80% | 82% | 85% |

As could be seen from the above data, after allisartan isoproxil pharmaceutical compositions obtained in the Example 1 to 10 were stored for 30 days at high temperature conditions (40 °C), the dissolution performance was not significantly reduced, so that we could conclude the solid dispersion in corresponding pharmaceutical composition did not show aging phenomenon significantly;
In contrast, Comparative Example 1 was the formulation of Example D5 disclosed in patent CN200880001668.0, despite preparation obtained in available technologies met the requirement of dissolution performance and stability, the mass of preparation (912mg) was quite larger, so patient compliance was low;
For Comparative Example 2, no surfactant was added as compared with Example 3, ageing resistance ability of corresponding preparation obtained in Comparative Example 2 decreased obviously, which could not meet the requirements medical use;
For Comparative Example 4, too less amount of surfactant was added compared to Example 5, although corresponding preparation showed certain aging resistance ability, the dissolution performance had very obvious change, making the quality of preparation fluctuated, which could not meet the requirements of medical use.

The above embodiments are preferable examples of the present invention, but the detailed description is not restricted by the examples; other change, modification, substitution, combination, simplification and any other departure from the spirit and principle of the present invention are considered as equivalent replacement, and should be included within the protection of the invention.

## Claims

1. An allisartan isoproxil solid dispersion composed of allisartan isoproxil and pharmaceutically acceptable carrier material, wherein the mentioned carrier material comprises solubilizing carrier, wherein mentioned allisartan isoproxil solid dispersion also includes surfactant, and the mass ratio of allisartan isoproxil to the surfactant is 1: 0.01 to 0.10.

2. An allisartan isoproxil solid dispersion according to claim 1, wherein the surfactant is selected from one or mixture of two or more of sodium lauryl sulfate, hexadecyl sulfate, octadecyl sulfate, lecithin, polyol type nonionic surfactants, fatty acid sorbitan, polysorbate, polyoxyethylene fatty alcohol ethers.

3. An allisartan isoproxil solid dispersion according to claims 1-2 , wherein the surfactant in mentioned allisartan isoproxil solid dispersion is selected from one or mixture of two or more of sodium lauryl sulfate, lecithin, sucrose stearate, span 20, tween 20, tween 80, Polyoxyl 40 Hydrogenated Castor Oil.

4. An allisartan isoproxil solid dispersion according to claims 1-3, wherein the mass ratio of allisartan isoproxil to the surfactant in mentioned allisartan isoproxil solid dispersion is 1: 0.02 to 0.06.

5. An allisartan isoproxil solid dispersion according to claims 1-4, wherein the mentioned solubilizing carrier in mentioned allisartan isoproxil solid dispersion is selected from one or mixture in any ratio of two or more of vinyl pyrrolidone homopolymer or copolymer, polyvinyl alcohol, polyethylene glycol, cellulose ethers, acrylic polymers, hydroxypropyl methylcellulose phthalate, cellulose acetate phthalate, hydroxypropyl methylcellulose cellulose acetate succinate.

6. An allisartan isoproxil solid dispersion according to claims 1-5, wherein the solubilizing carrier in mentioned allisartan isoproxil solid dispersion is one or mixture in any ratio of two or more of povidone, copovidone, PEG4000, PEG6000, hydroxypropyl cellulose, hydroxypropylmethyl cellulose, hydroxypropyl methyl cellulose phthalate.

7. An allisartan isoproxil solid dispersion according to claims 1-6, wherein the mass ratio of allisartan isoproxil to the solubilizing carrier in mentioned allisartan isoproxil solid dispersion is 1: 0.15 to 0.5.

8. An allisartan isoproxil solid dispersion according to claims 1-7, wherein the mass ratio of allisartan isoproxil to the solubilizing carrier in mentioned allisartan isoproxil solid dispersion is 1: 0.20 ∼ 0.30.

9. An allisartan isoproxil solid dispersion according to claims 1-8, wherein the mentioned allisartan isoproxil solid dispersion comprises excipient, and the mass ratio of allisartan isoproxil to excipient is 1: 0.10 to 1.00.

10. An allisartan isoproxil solid dispersion according to claim 9, wherein the mentioned the excipient in the present invention is one or mixture in any ratio of two or more of cross-linked povidone, cross-linked sodium carboxymethyl cellulose, low-substituted hydroxypropyl cellulose, sodium carboxymethyl starch, microcrystalline cellulose, starch, pre-gelatinized starch, lactose, dextrin, mannitol, calcium sulfate, calcium phosphate, calcium hydrogen phosphate.

11. An allisartan isoproxil solid dispersion according to claims 9-10, **characterized in that** the mentioned allisartan isoproxil solid dispersion comprises excipient, and the mass ratio of allisartan isoproxil to the excipients is 1:0.30 to 0.80.

12. An allisartan isoproxil pharmaceutical composition, wherein the mentioned pharmaceutical composition is composed of allisartan isoproxil solid dispersion according to claims 1-11 and pharmaceutically acceptable excipient.

13. An Allisartan isoproxil pharmaceutical composition according to claim 12, wherein the mentioned pharmaceutically acceptable excipient comprises one or mixture of two or more of disintegrant, binders, filler, lubricant; the mentioned disintegrant is one or mixture of two or more of crosslinked sodium carboxymethyl cellulose, cross-linked povidone, sodium carboxymethyl starch, low-substituted hydroxypropyl cellulose, starch, pre-gelatinized starch; the mentioned binder is one or mixture of two or more of hydroxypropyl methylcellulose, hydroxypropyl cellulose, sodium carboxymethyl cellulose, povidone, starch slurry, gelatin; and wherein the filler is one or mixture of two or more of lactose, mannitol, dextrin, microcrystalline cellulose, starch, pregelatinized starch, calcium sulfate, calcium phosphate, calcium hydrogen phosphate; the mentioned lubricant is one or mixture of two or more of stearic acid, magnesium stearate, colloidal silicon dioxide, talc, polyethylene glycol.

14. An allisartan isoproxil pharmaceutical composition according to claim 13, wherein the mass ratio of solid dispersion to the disintegrant in the pharmaceutical composition is 1: 0.02 to 0.20; the mass ratio of solid dispersion to the binder is 1: 0.01 to 0.05; the mass ratio of solid dispersion to the filler is 1:0.02 to 0.20.

15. An Allisartan isoproxil pharmaceutical composition according to claims 12-14, wherein the mentioned allisartan isoproxil pharmaceutical composition can be tablets, capsules, granules or pills.

16. An allisartan isoproxil pharmaceutical composition according to claims 12-15, wherein the allisartan isoproxil pharmaceutical composition can be used for the treatment of hypertension and its complications.
